# EUROPEAN PATENT SPECIFICATION

(11) **EP 0 573 628 B1**
(45) Date of publication and mention of the grant of the patent: **20.01.1999**
(21) Application number: 92925146.0
(22) Date of filing: 09.11.1992
(51) Int. Cl.: C07H 17/08, C12P 19/62, C12N 1/20, A01N 43/22

(54) **NEW A83543 COMPOUNDS AND PROCESS FOR PRODUCTION THEREOF**
A 83543 Verbindungen und Verfahren zu ihrer Herstellung
NOUVEAUX COMPOSES A83543 ET LEUR PROCEDE DE PRODUCTION

(30) Priority: 08.11.1991 US 790282; 08.11.1991 US 790287; 08.11.1991 US 790616
(43) Date of publication of application: 15.12.1993
(73) Proprietor: Dow AgroSciences LLC, Indianapolis, Indiana 46268 (US)
(72) Inventor: CREEMER, Lawrence, Indianapolis, IN 46234 (US); KIRST, Herbert, A., Indianapolis, IN 46278 (US); MYNDERSE, Jon, S., Indianapolis, IN 46208 (US); BROUGHTON, Mary, C., Indianapolis, IN 46220 (US); HUBER, Mary, L., B., Danville, IN 46122 (US); MARTIN, James, W., Coatesville, IN 46121 (US); TURNER, Jan, R., Carmel, IN 46032 (US)
(74) Representative: Raynor, John
(86) International application number: US9209684
(87) International publication number: WO9309126

(56) References cited:
- EP-A- 0 375 316
- JOURNAL OF THE AMERICAN CHEMICAL SOCIETY. vol. 114, no. 6, 11 March 1992, GASTON, PA US pages 2260 - 2 D.A.EVANS ET AL. 'Asymmetric Synthesis of the Macrolide (+)-A83543A (Lepicidin) Aglycon.'
- Chemistry of the Glycosidic Bond: Formation and Cleavage, A.F.Bochkow & G.E.Zaikov, Pergamon Press, 1979, pp183-184
- ANTIBIOTICS AND CHEMOTHERAPY, vol.11, no.5, , page 328 - 334, R.L.HAMILL ET. AL. 'TYLOSIN, A NEW ANTIBIOTIC: II. ISOLATION, PROPERTIES, AND PREPARATION OF DESMYCOSIN, A MICROBIOLOGICALLY ACTIVE DEGRADATION PRODUCT'
- TETRAHEDRON LETTERS, vol.34, no., 1964, page 2339 - 2345, R.B.MORIN & M.GORMAN 'THE PARTIAL STRUCTURE OF TYLOSIN, A MACROLIDE ANTIBIOTIC'
- TETRAHEDRON LETTERS, vol.54, no., 1970, page 4737 - 4740, R.B.MORIN, M.GORMAN, R.L.HAMILL & P.V.DEMARCO 'THE STRUCTURE OF TYLOSIN'
- Journal of the American Chemical Society, Vol.80, 1958, pp 3777-3782

## Description

The invention relates to new compounds of fermentation product A83543.

Target insects are rapidly developing resistance to synthetic insecticides, including the development of resistance to the newer pyrethroid insecticides (see Pickett (1988), Chem. Britain, 137). Therefore, new insecticides are in demand.

Fermentation product A83543, a family of related compounds produced by strains of *Saccharopolyspora spinosa,* was recently discovered and was shown to exhibit excellent insecticidal activity. A83543 and each of the compounds are useful for the control of mites and insects, particularly *Lepidoptera* and *Diptera* species.

By "A83543 compounds" is meant natural compounds consisting of a 5,6,5-tricylic ring system, fused to a 12-membered macrocyclic lactone, a neutral sugar and an amino sugar (see Kirst *et al.* (1991), Tetrahedron Letters, 32:4839). The family of natural A83543 compounds includes a genus taught in EPO Application No. 0375316 and having the following general formula: 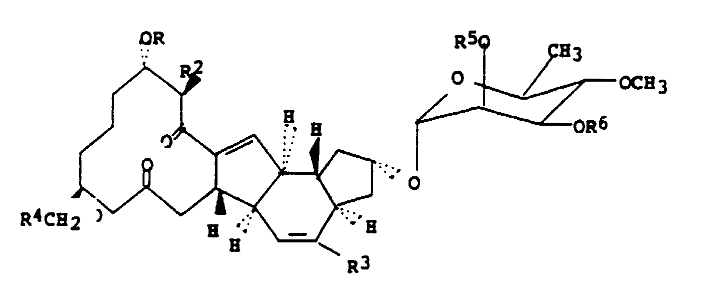 wherein R¹ is H or a group selected from 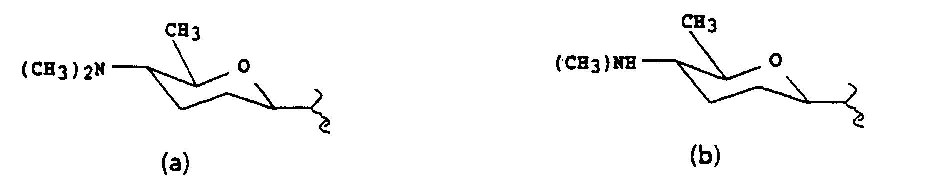 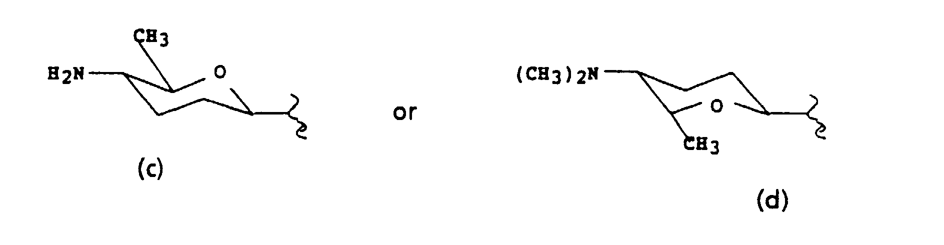 and R², R⁴, R³, R⁵ and R⁶ are hydrogen or methyl; or an acid addition salt thereof when R¹ is other than hydrogen.

The A83543 fermentation product has been shown to comprise individual compounds A83543A, A83543B, A83543C, A83543D, A83543E, A83543F, A83543G, A83543H and A83543J and various psuedoaglycones thereof (see European Patent Publication No. O 375 316). The structures of these individual compounds are shown below. 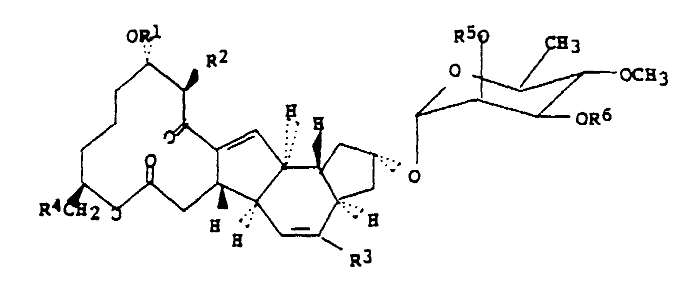 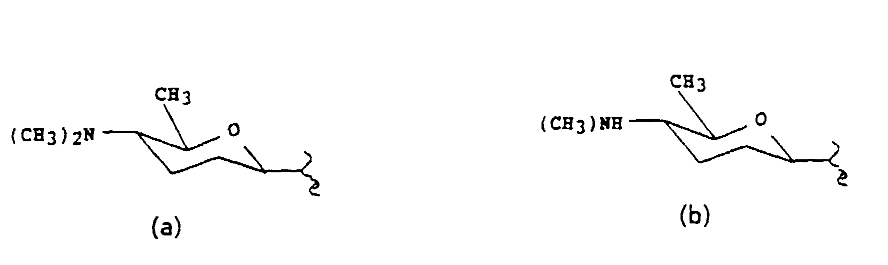 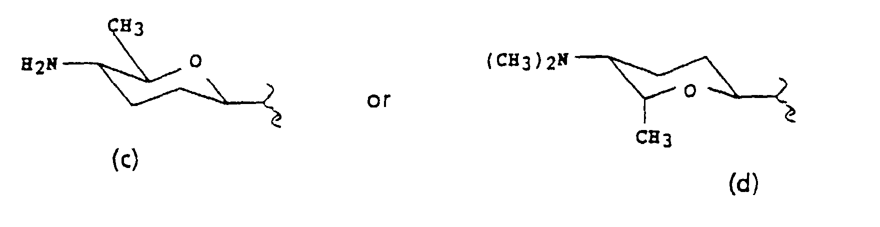 wherein R¹, R², R³, R⁴, R⁵ and R⁶ are for each compound as follows:

| Structures of A83543 Compounds | | | | | | |
|---|---|---|---|---|---|---|
| Compound | R¹ | R² | R³ | R4 | R⁵ | R6 |
| A | (a) | Me | H | Me | Me | Me |
| B | (b) | Me | H | Me | Me | Me |
| C | (c) | Me | H | Me | Me | Me |
| D | (a) | Me | Me | Me | Me | Me |
| E | (a) | Me | H | H | Me | Me |
| F | (a) | H | H | Me | Me | Me |
| G | (d) | Me | H | Me | Me | Me |
| R | (a) | Me | H | Me | H | Me |
| J | (a) | Me | R | Me | Me | H |
| PsaAl | H | Me | H | Me | Me | Me |
| PsaDl | H | Me | Me | Me | Me | Me |
| PsaEl | H | Me | H | H | Me | Me |
| PsaFl | H | H | H | Me | Me | Me |
| PsaHl | H | Me | R | Me | H | Me |
| PsaJl | H | Me | H | Me | Me | H |
| PsaLl | H | Me | Me | Me | Me | H |

The present invention is directed to compounds of Formula 1: 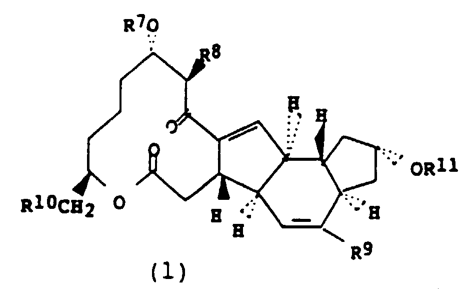 wherein R⁷, R⁸, R⁹ R¹⁰ and R¹¹ are each individually as follows:

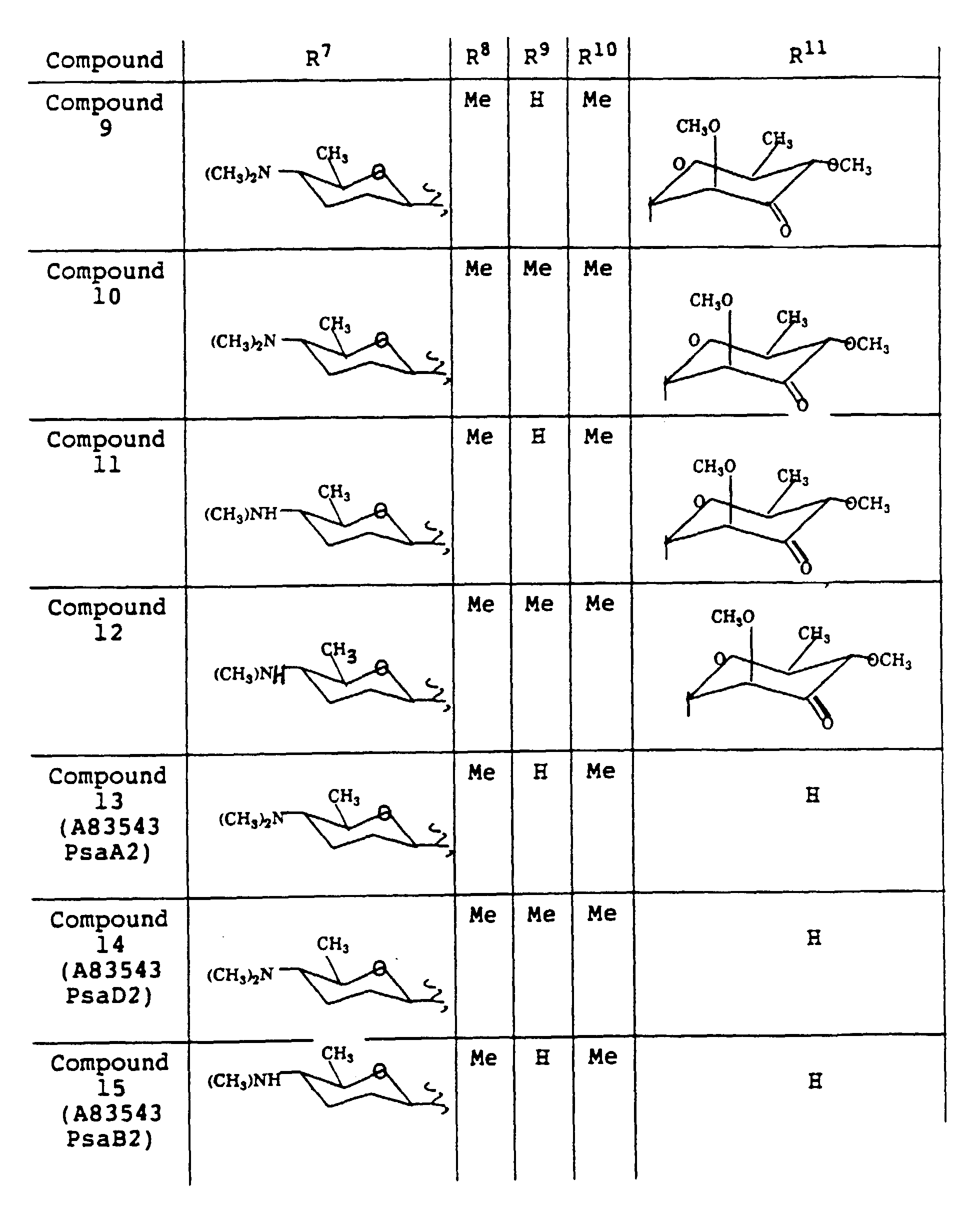

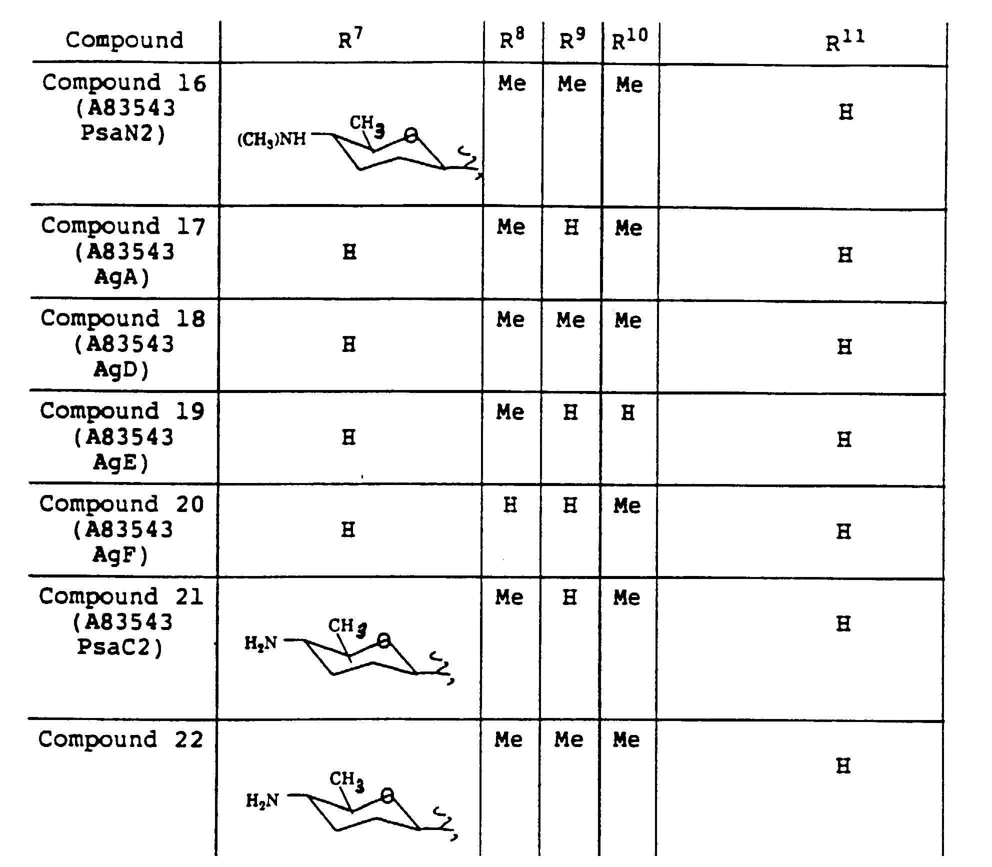

The invention is also directed to a process for preparing compounds 13, 14, 15, or 16 which process comprises removing the ketone-containing R" group from a corresponding compound 9, 10, 11 or 12.

Preferably, the above-mentioned process comprises oxidising a corresponding compound of formula 1 wherein R" is 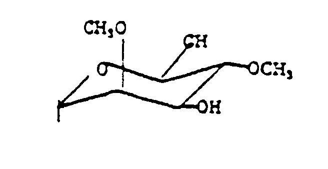 to produce the corresponding ketone-containing compound 9, 10, 11 or 12 and then removing the kenone-containing R" group from the said compound 9, 10, 11 or 12 to produce the corresponding compound 13, 14, 15 or 16.

Another aspect of the invention is a process for preparing a compound 21 or 22, which process comprises demethylating the corresponding compound 13 or 14.

The invention is further directed to a process for preparing a compound 9, 10, 11 or 12, which process comprises oxidising a corresponding compound of formula 1, wherein R" is

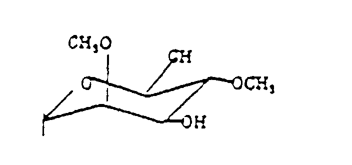

The pseudoaglycones of the present invention are produced by the following scheme:

### Scheme A

A83543J→Compound 9→Compound 13→Compound 17
A83543PsaA2→A83543AgA

A83543L→compound 10→Compound 14→Compound 18
A83543PsaD2→A83543AgD

A83543M→Compound 11→Compound 15→Compound 17
A83543PsaB2→A83543AgA

A83543N→Compound 12→Compound 16→Compound 18
A83543PsaN2→A83543AgD

A83543J is disclosed in EP-A-0375316. The starting materials A83543L, A83543M and A83543N are compounds of Formula 1 wherein R⁷, R⁸, R⁹, R¹⁰ and R¹¹ are each individually as follows:

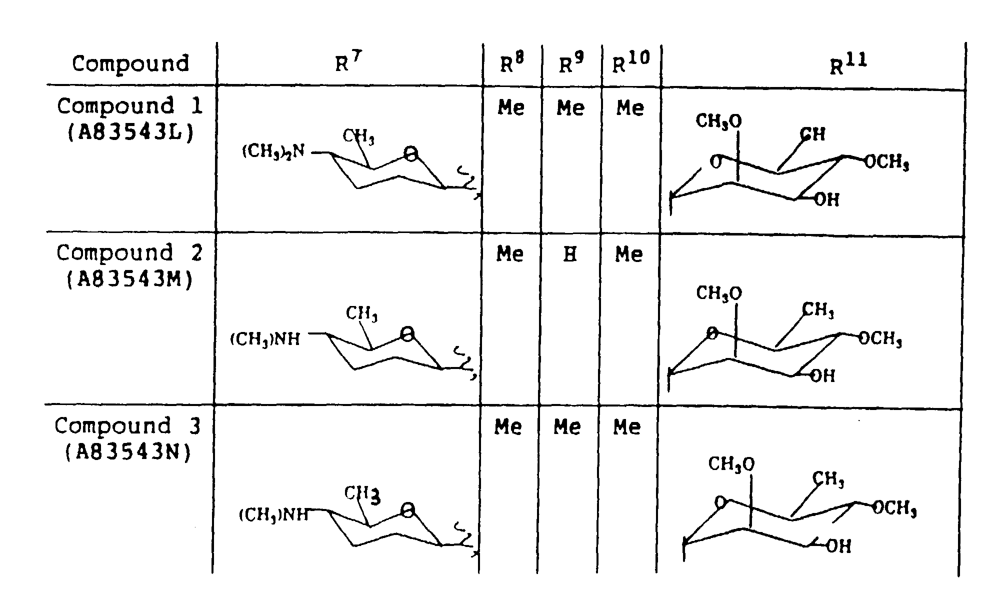

In another one of its aspects, the invention provides a process for producing A83543AgA, A83543AgD, A83543AgE, or A83543AgF, which comprises
(a) hydrolyzing A83543A, A83543B, A83543C, A83543G, A83543H, A83543J, A83543PsaA1, A83543PsaA2, A83543PsaH1, A83543PsaJ1, A83543PsaB2, or A83543PsaC2 to produce A83543AgA; or
(b) hydrolyzing A83543PsaD2 or A83543PsaN2 to produce A83543AgD; or
(c) hydrolyzing A83543E or A83543PsaE1 to produce A83543AgE; or
(d) hydrolyzing A83543F or A83543PsaF1 to produce A83543AgF.

The pseudoaglycones and aglycones of this invention are useful as intermediates in the preparation of insecticides. For example, when appropriate microorganisms are cultured in the presence of the claimed compounds, the claimed compounds are bioconverted to insecticidally active A83543 components, as illustrated in Example 19.

Glycosylation may be carried out by chemical synthesis as well as by microbial bioconversion.

The chemical structures of the starting materials, Compounds 1-3 were determined by spectrometric methods, including nuclear magnetic resonance spectroscopy (NMR), and ultraviolet spectroscopy (UV), and by comparison to the known A83543 compounds (see Kirst, *et al.* (1991), *supra).* The following paragraphs describe the physical and spectral properties of Compounds 1-3.

### A83543L:

A83543L has the following characteristics:
Molecular weight: 731
Empirical formula: C₄₁H₆₅NO₁₀
UV (EtOH): 244 nm (ε=10,362)
MS (FAB): (M+H) m/z 732

Table I summarizes the ¹H and ¹³C nuclear magnetic resonance (NMR) spectral data for A83543L (in d₆-acetone).

**Table I.**

| ^{**1**}**H and** ^{**13**}**C NMR data of A83543L in acetone-d**_{**6**} | | |
|---|---|---|
| Position | ¹³C | ¹H* |
| 1 | 172.59 | -- |
| 2 | 34.32 | 3.07/2.42 |
| 3 | 48.39 | 2.91 |
| 4 | 42.75 | 3.45 |
| 5 | 123.29 | 5.53 |
| 6 | 137.19 | -- |
| 7 | 45.33 | 2.18 |
| 8 | 35.61 | 2.01/1.45 |
| 9 | 76.62 | 4.32 |
| 10 | 38.59 | 2.36/1.38 |
| 11 | 46.99 | 1.03 |
| 12 | 49.99 | 2.77 |
| 13 | 148.51 | 7.02 |
| 14 | 145.11 | -- |
| 15 | 203.09 | -- |
| 16 | 48.43 | 3.30 |
| 17 | 80.91 | 3.55 |
| 18 | 35.04 | 1.55 |
| 19 | 22.50 | 1.79/1.19 |
| 20 | 30.89 | 1.53 |
| 21 | 76.81 | 4.45 |
| 22 | 29.11 | 1.49 |
| 23 | 9.55 | 0.81 |
| 24 | 16.29 | 1.13 |
| 6-CH₃ | 20.85 | 1.74 |
| 1' | 96.37 | 4.86 |
| 2' | 82.44 | 3.33 |
| 3' | 72.33 | 3.73 |
| 4' | 84.59 | 2.95 |
| 5' | 68.34 | 3.50 |
| 6' | 18.31 | 1.20 |
| 2'-OCH₃ | 59.05 | 3.44 |
| 4'-OCH₃ | 60.74 | 3.51 |
| 1" | 104.06 | 4.46 |
| 2" | 31.90 | 1.93/1.39 |
| 3" | 18.74 | 1.83/1.52 |
| 4" | 65.97 | 2.12 |
| 5" | 74.04 | 3.57 |
| 6" | 19.39 | 1.21 |
| N(CH₃)₂ | 40.97 | 2.21 |

| | | |
|---|---|---|
| * Values were taken from a heteronuclear one bond 2D correlation spectrum. | | |

### A83543M:

A83543M has the following characteristics:
Molecular weight: 703
Empirical formula: C₃₉H₆₁NO₁₀
UV (EtOH): 244 nm (ε=10,240)
MS (FAB): (M+H) m/z 704

Table II summarizes the ¹H and ¹³C nuclear magnetic resonance (NMR) spectral data for A83543M (in d₆-acetone).

**Table II.**

| ^{**1**}**H and** ^{**13**}**C NMR data of A83543M in acetone-d**_{**6**} | | |
|---|---|---|
| Position | ¹³C | ¹H* |
| 1 | 172.65 | -- |
| 2 | 34.53 | 3.08/2.44 |
| 3 | 48.80 | 2.94 |
| 4 | 42.36 | 3.50 |
| 5 | 129.80 | 5.87 |
| 6 | 130.34 | 5.91 |
| 7 | 42.05 | 2.14 |
| 8 | 37.21 | 1.97/1.36 |
| 9 | 77.04 | 4.34 |
| 10 | 38.30 | 2.36/1.36 |
| 11 | 47.12 | 0.94 |
| 12 | 50.44 | 2.87 |
| 13 | 148.34 | 7.05 |
| 14 | 144.93 | -- |
| 15 | 203.08 | -- |
| 16 | 48.36 | 3.31 |
| 17 | 81.22 | 3.55 |
| 18 | 35.15 | 1.52 |
| 19 | 22.38 | 1.78/1.17 |
| 20 | 31.09 | 1.50 |
| 21 | 76.82 | 4.66 |
| 22 | 29.11 | 1.48 |
| 23 | 9.57 | 0.80 |
| 24 | 16.44 | 1.13 |
| 1' | 96.46 | 4.84 |
| 2' | 82.50 | 3.31 |
| 3' | 72.23 | 3.72 |
| 4' | 84.61 | 2.94 |
| 5' | 68.40 | 3.48 |
| 6' | 18.34 | 1.19 |
| 2'-OCH₃ | 59.08 | 3.44 |
| 4'-OCH₃ | 60.72 | 3.50 |
| 1" | 104.15 | 4.48 |
| 2" | 31.78 | 1.88/1.42 |
| 3" | 29.11 | 2.11/1.23 |
| 4" | 61.86 | 2.02 |
| 5" | 76.55 | 3.27 |
| 6" | 19.34 | 1.22 |
| N(CH₃)₂ | 34.16 | 2.34 |

| | | |
|---|---|---|
| * Values were taken from a heteronuclear one bond 2D correlation spectrum. | | |

### A83543N:

A83543N has the following characteristics:
Molecular weight: 717
Empirical formula: C₄₀H₆₃NO₁₀
UV (EtOH): 244 nm (ε=10,446)
MS (FAB): (M+H) m/z 718

Table III summarizes the ¹H and ¹³C nuclear magnetic resonance (NMR) spectral data for A83543N (in d₆-acetone).

**Table III.**

| ^{**1**}**H and** ^{**13**}**C NMR data of A83543N in acetone-d**_{**6**} | | |
|---|---|---|
| Position | ¹³C | ¹H* |
| 1 | 172.65 | -- |
| 2 | 34.41 | 3.06/2.43 |
| 3 | 50.00 | 2.90 |
| 4 | 42.85 | 3.45 |
| 5 | 123.38 | 5.55 |
| 6 | 137.25 | -- |
| 7 | 45.39 | 2.18 |
| 8 | 35.67 | 2.01/1.46 |
| 9 | 76.78 | 4.32 |
| 10 | 38.65 | 2.37/1.40 |
| 11 | 47.07 | 1.03 |
| 12 | 50.07 | 2.78 |
| 13 | 148.41 | 7.03 |
| 14 | 145.17 | -- |
| 15 | 203.14 | -- |
| 16 | 48.44 | 3.31 |
| 17 | 81.14 | 3.56 |
| 18 | 35.13 | 1.51 |
| 19 | 22.44 | 1.81/1.20 |
| 20 | 31.05 | 1.51 |
| 21 | 76.78 | 4.65 |
| 22 | 29.11 | 1.48 |
| 23 | 9.56 | 0.81 |
| 24 | 16.41 | 1.12 |
| 6-CH₃ | 20.82 | 1.73 |
| 1' | 96.51 | 4.86 |
| 2' | 82.51 | 3.32 |
| 3' | 72.24 | 3.73 |
| 4' | 84.62 | 2.95 |
| 5' | 68.41 | 3.50 |
| 6' | 18.34 | 1.18 |
| 2'-OCH3 | 59.09 | 3.43 |
| 4'-OCH₃ | 60.71 | 3.51 |
| 1" | 104.13 | 4.48 |
| 2" | 31.78 | 1.87/1.40 |
| 3" | 29.11 | 2.11/1.23 |
| 4" | 61.88 | 2.00 |
| 5" | 76.58 | 3.26 |
| 6" | 19.33 | 1.22 |
| N(CH₃)₂ | 34.18 | 2.34 |

| | | |
|---|---|---|
| * Values were taken from a heteronuclear one bond 2D correlation spectrum. | | |

Compounds 1-3, natural A83543 components, are generally produced by culturing a suitable A83543-producing strain of *S. spinosa* sp. nov. under submerged aerobic conditions in a desired culture medium, until a recoverable amount of a natural factor is produced. Compounds 1-3 can be recovered using various isolation and purification procedures which are understood in the art.

A83543M and A83543N may also be prepared by chemical demethylation from A83543J and A83543L.

The N-demethyl derivatives are prepared by reacting a natural factor in the presence of between one and thirteen equivalents of iodine and a suitable base such as sodium acetate. The reaction is carried out in a polar organic solvent, such as methanol, or a mixture of a polar organic solvent and water, such as aqueous methanol. The reaction is preferably carried out at a temperature of from between 30°C to 90°C for between 2 to 6 hours, at a pH of between 8 and 10.

In addition, A83543L and A83543N may be prepared by culturing any of the known A83543-producing strains in the presence of A83543PsaL1.

Compounds 1-3 can react to form various acid addition salts. Representative suitable salts include those salts formed by standard reactions with both organic and inorganic acids such as, for example, sulfuric, hydrochloric, phosphoric, acetic, succinic, citric, lactic, maelic, fumaric, cholic, pamoic, mucic, glutamic, camphoric, glutaric, glycolic, phthalic, tartaric, formic, lauric, stearic, salicyclic, methanesulfonic, benzenesulfonic, sorbic, picric, benzoic, cinnamic, and like acids. These salts are useful, for example, in separating and purifying Compounds 1-3. In addition, some of the salt forms may have increased water solubility. These salts are prepared using standard procedures for salt preparation.

For convenience in the discussions which follow regarding the production of Compounds 1-3 by cultivating A83543- producing strains of S. spinosa, two known A83543A-producing strains have been given the following designations: A83543.1 and A83543.4 (see EPO No. 0 375 316); as discussed below these strains have been used to develop new strains. Two new A83543J-producing strains have been given the designation A83543.6 and A83543.7; components A83543L, A83543M and A83543N are produced by A83543.6 and A83543.7. Cultures A83543.1, A83543.4, A83543.6 and A83543.7 have been deposited and made a part of the stock culture collection of the Midwest Area Regional Research Center, Agricultural Research Service, United States Department of Agriculture, from which they are available to the public under the following accession numbers:

| NRRL No. | Strain No. |
|---|---|
| 18395 | A83543.1 |
| 18538 | A83543.4 |
| 18719 | A83543.6 |
| 18720 | A83543.7 |

Culture A83543.1 was obtained by chemical mutation of culture A83543, which was isolated from a soil sample collected in the Virgin Islands (see Mertz and Yao (1990), Int'1 J. of Systematic Bacteriology, 40:34). Cultures A83543.4 and A83543.6 were derived from culture A83543.1 by chemically-induced mutagenesis with N-methyl-N'-nitro-N-nitrosoguanidine. Culture A83543.7 was derived from A83543.4 by chemically-induced mutagenesis with N-methyl-N'-nitro-N-nitrosoguanidine. The following data show that these distinct isolates are all strains of *S. spinosa* and have very few cultural, morphological or biochemical differences. Except for differences in the production of the A83543 components, these isolates appear similar to the parent culture.

### Cultural Characteristics

Cultures A83543.1, A83543.4, A83543.6 and A83543.7 were grown on twelve agar plating media and compared for growth, reverse color, aerial hyphae production, spore mass color, and soluble pigment production. No significant differences were observed on any of the media used. The cultures grew well on both complex and defined media. Aerial hyphae were produced on most of the media used. The aerial spore mass color was predominantly white, and the reverse side was yellow to yellow-brown. No distinctive pigmentation was present; however, a soluble brown pigment was released into some media. These cultural characteristics are the same as presented in the original taxonomic description of A83543.1 (see Mertz and Yao (1990), *supra).*

### Morphological Characteristics

No significant differences were observed between any of the strains compared. Well-formed aerial hyphae, which were segmented into long chains of spores arranged as hooks and open loops, were present on most of the media. Spirals were also observed, but they were short and incomplete. The general morphology was rectus-flexibilis. Aerial hyphae of each of the strains had a distinctive bead-like appearance, with many empty spaces in the spore chain. This feature demonstrated that a spore sheath encased the spore chain, which is a distinctive feature of the genus *Saccharopolyspora.*

### Physiological Characteristics

Fatty acid analyses from each of the strains were compared. Cells were grown for 96 hours at 28°C in trypticase soy broth (Difco Laboratories, Detroit, MI). Fatty acid methyl esters were analyzed by gas-liquid chromatography with a model 5898A computer-controlled gas-liquid chromatography system (Hewlett-Packard Co., Palo Alto, CA) (see Miller and Berger, "Bacterial Identification by Gas Chromatography of Whole Cell Fatty Acids," Hewlett-Packard Application Note 228-41. These results are presented in Table IX).

**TABLE IX.**

| Percentage Fatty Acid Composition of A83543 Strains | | | | |
|---|---|---|---|---|
| **Fatty Acid** | **A83543.1** | **A83543.4** | **A83543.6** | **A83543.7** |
| 15:0 ISO | 15.95 | 22.47 | 16.49 | 17.00 |
| 16:0 ISO | 28.71 | 22.00 | 25.76 | 27.39 |
| 16:1 Cis 9 | -- | 1.35 | -- | -- |
| 15:0 ISO 20H | 2.67 | 2.02 | 3.87 | 3.95 |
| 16:0 | 1.20 | 0.69 | 0.63 | 0.60 |
| 17:1 ISO F¹ | 5.52 | 8.62 | 7.54 | 5.51 |
| 17:0 Iso | 13.55 | 20.67 | 16.40 | 13.89 |
| 17:0 Anteiso | 8.39 | 3.94 | 4.69 | 5.18 |
| 17:1 B | 4.14 | 3.97 | 4.65 | 6.68 |
| 17:1 C | 2.52 | 2.88 | 4.90 | 5.53 |
| 17:0 | 4.26 | 1.49 | 3.13 | 3.84 |
| 16:1 20H | 1.87 | 1.52 | 1.93 | 0.92 |
| 18:1 Iso F | 6.55 | 4.16 | 5.82 | 6.00 |
| 18:1 Cis 9 | 0.34 | 1.03 | 0.64 | 0.63 |

| | | | | |
|---|---|---|---|---|
| ¹F, B and C indicate double bond positions or configurations that are unknown. | | | | |

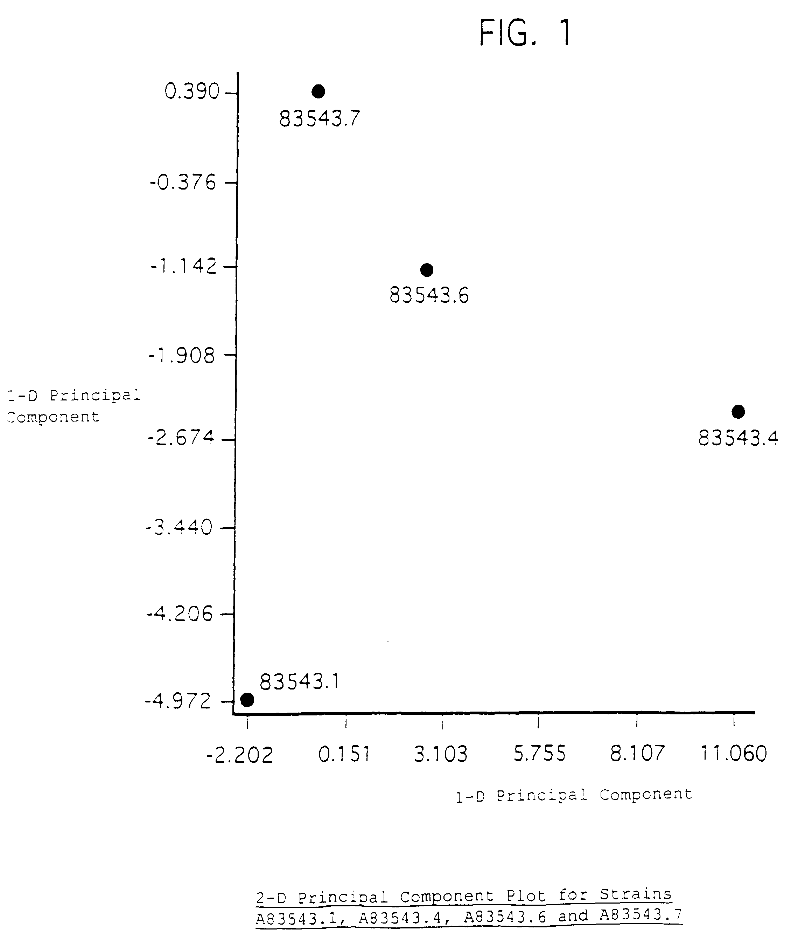

Principal-component analysis is a branch of multivariate statistics that deals with internal relationships of a set of variables. In this analysis, the greatest amount of variance within the original data or test results is expressed as principal components (see Alderson, "The Application and Relevance of Nonheirarchic Methods in Bacterial Taxonomy", in Computer-Assisted Bacterial Systematics 227 (1985)). A plot showing scatter or variability can be constructed. Relationships can be evaluated by examining the variance, and a microbial population characterized. A two-dimensional principal component plot from the fatty acid analyses of strains A83543.1, A83543.4, A83543.6 and A83543.7 is shown in Figure 1. The values refer to the degrees of separation between the strains involved. The differences between the strains represent strain differences.

As is the case with other organisms, the characteristics of the A83543A-producing and the A83543J-producing strains are subject to variation. Thus, mutants of these strains may be obtained by physical and chemical methods known in the art. For example, other strains may be obtained by treatment with chemicals such as N-methyl-N'-nitro-N-nitrosoguanidine.

Compounds 1-3 are produced by culturing an A83543-producing strain of *S. spinosa,* selected from the group consisting of NRRL 18719 and NRRL 18720, or an A83543J-producing mutant thereof, in a suitable culture medium. An "A83543J-producing mutant" is a natural or induced mutant derived from *S. spinosa* NRRL 18719 or NRRL 18720 which is capable of producing recoverable amounts of A83543J (as well as A83543L, A83543M or A83543N).

After production, Compounds 1-3 may be separated from the culture medium using various isolation and purification procedures which are well understood in the art. For economy in production, optimal yield, and ease of product isolation, certain culture media are preferred. For example, preferred carbon sources in large-scale fermentation are glucose and methyl oleate, although ribose, xylose, fructose, galactose, mannose, mannitol, soluble starch, potato dextrin, oils such as soybean oil and the like can also be used. Preferred nitrogen sources are cottonseed flour, peptonized milk and corn steep liquor, although fish meal, digested soybean meal, yeast extract, enzyme-hydrolyzed casein, beef extract, and the like can also be used. Among the nutrient inorganic salts which can be incorporated in the culture media are the customary soluble salts capable of yielding zinc, sodium, magnesium, calcium, ammonium, chloride, carbonate, sulfate, nitrate and like ions. Essential trace elements necessary for the growth and development of the organism should also be included in the culture medium. Such trace elements commonly occur as impurities in other substituents of the medium in amounts sufficient to meet the growth requirements of the organism.

Usually, if foaming is a problem, small amounts (i.e., 0.2 ml/L) of an antifoam agent such as polypropylene glycol may be added to large-scale fermentation media. In the case of the A83543-producing cultures, however, conventional defoamers inhibit A83543 production. Foaming can be controlled by including soybean oil or PLURONIC L-101 (BASF, Parsippany, NJ) in the medium (1-3%). Additional oil may be added if foaming develops.

For production of substantial quantities the natural factors, submerged aerobic fermentation in stirred bioreactors is preferred; however, small quantities of natural factors may be obtained by shake-flask culture. Because of the time lag in production commonly associated with inoculation of large bioreactors with the spore form of the organism, it is preferable to use a vegetative inoculum. The vegetative inoculum is prepared by inoculating a small volume of culture medium from a stock culture preserved in liquid nitrogen to obtain a fresh, actively growing culture of the organism. The vegetative inoculum is then transferred to a larger bioreactor. The vegetative inoculum medium can be the same as that used for larger fermentations, but other media are also suitable.

Compounds 1-3 are produced by A83543J-producing strains when grown at temperatures between about 24° and about 33°C. Optimum temperatures for production appear to be about 28-30°C.

As is customary in submerged aerobic culture processes, sterile air is blown into the vessel from the bottom while the medium is stirred with conventional turbine impellors. in general, the aeration rate and agitation rate should be sufficient to maintain the level of dissolved oxygen at or above 80% of air saturation, preferably above 70%, with an internal vessel pressure of about 0.34 atmospheres.

Production of Compounds 1-3 can be followed during the fermentation by testing extracts of the broth. A preferred method for following the production is analysis of the broth extracts by high performance liquid chromatography (HPLC). A suitable system for analysis is described in Example 1.

Following the production in shake flasks or in stirred reactors, Compounds 1-3 can be recovered from the fermentation medium by methods used in the art. The compounds produced during fermentation of the A83543J-producing strain occur in both the mycelia and the broth. Compounds 1-3 are lipophilic; when a substantial amount of oil is used in the fermentation, whole broth extraction is more efficient. If only small amounts of oil are used, the major portion of the Compounds 1-3 is present in the mycelia. In that case, more efficient recovery of Compounds 1-3 is accomplished by initially filtering the medium to separate the broth from the mycelial mass (the biomass).

Compounds 1-3 can be recovered from the biomass by a variety of techniques. A suitable technique involves washing the separated biomass with water to remove remaining broth, mixing the biomass with a polar solvent in which Compounds 1-3 is soluble, e.g., methanol or acetone, separating and concentrating the solvent, extracting the concentrate with a non-polar solvent and/or adsorbing it onto a reverse-phase silica gel adsorbent, such as reverse phase C₈ or C₁₈ resin, or a high porous polymer such as HP-20 or HP-20SS (Mitsubishi Chemical Industries Co., Ltd., Japan). The active material is eluted from the adsorbent with a suitable solvent such as, for example, acetonitrile:methanol mixtures, optionally containing small amounts of THF.

A preferred technique for isolating Compounds 1-3 from the biomass involves adding an equal volume of acetone to the whole broth, filtering the mixture in a ceramic filter to remove the biomass, and extracting the filtrate with ethyl acetate. The ethyl acetate extract is concentrated *in vacuo* to remove the acetone, and the aqueous layer is separated from the organic layer. The ethyl acetate solution is further concentrated *in vacuo,* and the concentrate is extracted with dilute aqueous acid (pH 3). Compounds 1-3 may be further purified by chromatography as described herein.

A more preferred technique for isolating Compounds 1-3 from the biomass involves adding an equal volume of acetone to the whole broth, filtering the mixture in a ceramic filter to remove the biomass, and adjusting the pH of the filtrate to about pH 9 to about pH 13. This solution is applied to HP-20SS (Mitsubishi Chemical Industries Co., Ltd., Japan) and the column washed with a mixture of methanol, acetonitrile, and water (1:1:2). Any one of Compounds 1-3 may be eluted with a 95:5 mixture of methanol/acetonitrile (1:1) and aqueous 0.1% ammonium acetate (pH 8.1). The fractions containing Compounds 1-3 are combined and lyophilized. Compounds 1-3 may be further purified by chromatography as described herein.

Alternatively, the culture solids, including medium constituents and mycelium, can be used without extraction or separation, but preferably after removal of water, as a source of Compounds 1-3. For example, after production of Compounds 1-3, the whole fermentation broth can be dried by lyophilization, by drum-drying, or by azeotropic distillation and drying.

### Example 1

### Assay Method for Compound 1 (A83543L), Compound 2 (A83543M) and Compound 3 (A83543N).

The following analytical high performance liquid chromatography (HPLC) method is useful for monitoring a fermentation for the production of Compound 1 (A83543L), Compound 2 (A83543M) and Compound 3 (A83543N) and other A83543 components:

A sample of the whole broth is diluted with three volumes of acetonitrile to extract the components from the mycelia. The resulting solution is then filtered through a 0.45 micron PTFE filter to remove particulate matter prior to injection into the HPLC assay system. A solution of purified A83543A at a concentration of 100 mg/ml in methanol is used as an external standard for the assay and peak areas of all A83543 components are related back to this calibration standard to determine concentrations of individual components.

### HPLC System:

Column Support: 4.6 x 100-mm column, ODS-AQ, 5µ spherical particles, 120Å pore (YMC, Inc., Morris Plains, NJ)
Mobile Phase: CH₃CN/MeOH/H₂O (40/40/20) containing 0.05% ammonium acetate
Flow Rate: 3 ml/min
Detection: UV at 250 nm
Retention Times:
   A83543A 9.1 min
   A83543J 5.7 min
   A83543L 7.3 min
   A83543M 2.6 min
   A83543N 3.3 min

### Example 2

Preparation of A83543J, Compound 1 (A83543L), Compound 2 (A83543M) and Compound 3 (A83543N) with Culture A83543.6

### A. Shake-flask Fermentation

The culture *Saccharopolyspora spinosa* NRRL 18719, either as a lyophilized pellet or as a suspension maintained in liquid nitrogen, was used to inoculate a vegetative medium having the following composition:

| **Vegetative Medium** | |
|---|---|
| **Ingredient** | **Amount (g)** |
| Trypticase broth* | 30 |
| Yeast extract | 3 |
| MgS0₄ · 7H₂0 | 2 |
| Glucose | 5 |
| Maltose | 4 |
| Deionized water | q.s. 1 L |
| Autoclave 30 min at 120°C | |

| | |
|---|---|
| * Baltimore Biological Laboratories, Cockeysville, MD | |

Slants or plates can be prepared by adding 2.5% agar to the vegetative medium. The inoculated slant is incubated at 30°C for 10 to 14 days. The mature slant culture is scraped with a sterile tool to loosen the spores and to remove and macerate the mycelial mat. One-fourth of the loosened spores and culture growth thus obtained is used to inoculate 50 ml of a first-stage vegetative medium. Alternatively, the first-stage medium may be inoculated from a liquid nitrogen ampoule.

When the culture is maintained in liquid nitrogen, ampoules are prepared by homogenizing a vegetative culture (48-72 hours incubation, 30°C), diluting 1:1 (volume:volume) with a sterile suspending agent and dispensing into sterile tubes (1.5 ml/tube). The suspending agent contains lactose (100 g), glycerol (200 ml), and deionized water (q.s. to 1 L).

A liquid nitrogen ampoule is used to inoculate 100 ml of vegetative medium in 500-ml Erlenmeyer flasks (or 50 ml of medium in 250-ml flasks). The cultures are incubated at 30°C for 48 hours on a shaker orbiting in a two-inch (5.08 cm) circle at 260 rpm.

The incubated culture (10% v/v inoculum) is used to inoculate 50 ml or 100 ml, dependent on the size of the Erlenmeyer flask, of a production medium having the following composition:

| **Production Medium** | |
|---|---|
| **Ingredient** | **Amount (g)** |
| Glucose | 80 |
| Peptonized milk* | 20 |
| Cottonseed flour** | 30 |
| Corn steep liquor | 10 |
| CaCO₃ (tech. grade) | 5 |
| Methyl oleate | 30*** |
| Tap water | q.s. to 1 L |
| pH adjusted to pH 7.0 with 1N NaOH, sterilized 40 min. at 120°C | |

| | |
|---|---|
| * Peptonized Milk Nutrient, Sheffield Products, Norwich, NY | |
| ** Proflo, Traders Protein, Memphis, TN | |
| ***The amount of methyl oleate was 30 ml | |

The inoculated production medium is incubated in 250-ml or 500-ml Erlenmeyer flasks at 30°C for 7 to 10 days on a shaker orbiting in a two-inch circle at 260 rpm.

### B. Stirred Reactor Fermentation

In order to provide a larger volume of inoculum, 10 ml of incubated first stage medium, prepared as described in Example 2, Section A, is used to inoculate 400 ml of a second-stage vegetative medium having the same composition as that of the first-stage medium. This second-stage vegetative medium is incubated in a 2 L wide-mouth Erlenmeyer flask for about 48 hours at 30°C on a shaker orbiting in a two-inch circle at 260 rpm. Incubated second-stage vegetative medium (2 L) thus prepared is used to inoculate 80 to 115 liters of sterile production medium, prepared as described in Example 2, Section A.

The inoculated production medium is allowed to ferment in a 165 L stirred bioreactor for 7 days to 10 days at a temperature of 30°C. The air-flow and agitator speed in the stirred vessel are computer controlled to maintain a dissolved oxygen level at or above 60% to about 80% of air saturation.

### Example 3

### Preparation of A83543J, A83543L, A83543M, and A83543N with Culture A83543.7.

The culture *Saccharopolyspora spinosa* NRRL 18720 may be used as described in Example 2 to prepare A83543J, A83543L, A83543M, and A83543N.

### Example 4

### Isolation of A83543J, A83543L, A83543M, and A83543N

Fermentation broth (105 L), prepared as described in Example 2, was adjusted to pH 10 (initially pH 6.8) by adding 5N NaOH. The resulting mixture was filtered through a ceramic filter. The filtrate was discarded, a mixture of acetone and water (1:1, 50 L) was added to the mycelial solids, and the resulting mixture was filtered. A second mixture of acetone and water (1:1, 50 L) was added to the mycelial solids, and the pH of the resulting mixture was adjusted to pH 3.0 with 25% sulfuric acid. The resulting mixture was filtered, and a third mixture of acetone and water (1:1 50 L) was added to the mycelial solid. The resulting mixture was filtered and the acidic filtrates were combined.

The combined filtrates were extracted with heptane (10 L). The phases were separated and the aqueous phase added to a second portion of heptane (10 L). The pH of the resulting mixture was adjusted to pH 10 with 5N NaOH. The resulting emulsion was diluted with 50 L of water. The phases were separated and the aqueous phase extracted with a third portion of heptane (10 L). The phases were separated and the second and third heptane extracts were combined and concentrated to a volume of about 4 liters. Upon standing, the concentrate separated into 3 phases: aqueous, emulsion, and organic. The organic phase was lyophilized to give 15.29 g of crude product.

The crude product was dissolved in methanol (500 ml), filtered, and concentrated to dryness *in vacuo.* The residue was dissolved in a second portion of methanol (20 ml) and applied to a column of LH-20 SEPHADEX (Pharmacia LKB Biotechnology, Inc., Piscataway, NJ, 7.5 cm x 46 cm), eluting with methanol and collecting 25 ml fractions. Using the HPLC system described in Example 1, the fractions were analyzed to determine which fractions contained the compounds of interest. Fractions 18-50 were combined and concentrated to dryness.

The residue was dissolved in a mixture of ethanol, acetonitrile, and water (5:5:1) and chromatographed in 1 ml portions on a preparative reverse-phase HPLC column (Rainin DYNAMAX-60A, C18, 41.4 mm x 300 mm, 8 mm particles, 60Å pore, Woburn, MA). The column was eluted with a mixture of methanol, acetonitrile and water (87.5:87.5:25) with ammonium acetate added to a final concentration of 0.1% (pH 7.6). The fractions were analyzed using an HPLC system, similar to that as described in Example 1, combining like fractions and concentrating to give three semi-pure concentrates A, B, and C.

Semi-pure concentrate C was rechromatographed on the system described in the preceding paragraph, loading 200 ml on each of 10 runs. The fractions from each of the runs were combined and concentrated to give preparations C1 and C2. Preparation C2 was chromatographed a third time; however, water was used in place of the 0.1% ammonium acetate (desalting step). Fractions containing A83543L in at least 99.5% HPLC purity were combined and concentrated. The residue was crystallized from ethanol/water (1:1) to give 2.4 g of A83543L.

Preparation C1 and semi-pure concentrate B were combined and desalted as described in the preceding paragraph (12 x 200 ml runs); however, the desired compound was eluted with a mixture of methanol, acetonitrile, and water (11:11:3). The fractions containing A83543J in at least 99.5% HPLC purity were combined and concentrated. The residue was dissolved in hot t-butanol and lyophilized to give 4.3 g of A83543J.

Semi-pure concentrate A was chromatographed as described above, except the desired compounds were eluted with a mixture of methanol, acetonitrile, and water (37.5:37.5:25), with ammonium acetate added to final concentration of 0.1%. The fractions from each of the runs (4) were combined and concentrated to give preparations A1, A2, and A3.

Preparation A1 was chromatographed using the column described above; however, the column was eluted with a mixture of methanol, acetonitrile, and water (2:2:1). Fractions containing A83543M in at least 99.5% HPLC purity were combined and concentrated. The residue was dissolved in t-butanol and lyophilized to give 136 mg of A83543M.

Preparation A2 was chromatographed and processed as described in the preceding paragraph to give 71 mg of A83543N.

### Example 5

### Synthesis of A83543M (Compound 2)

A83543J (105.4 mg, 0.15 mmol) and sodium acetate trihydrate (144.6 mg, 1.06 mmol) were added to a mixture of methanol and pH 9 buffer solution (Fisher Scientific, Lexington, MA). The resulting suspension was heated to 47°C, and then iodine (46.6 mg, .18 mol) was added in one portion. After approximately 10 min., the solution became homogeneous. After four hours at 47°C, the reaction was added to a 5% sodium thiosulfate solution. The resulting colorless aqueous mixture was extracted with methylene chloride. The methylene chloride extracts were combined, washed with brine, and dried over K₂CO₃. The dried methylene chloride solution was evaporated to dryness *in vacuo* to give 57.3 mg of A83543M as a pale yellow glass (54% yield).

### Example 6

### Synthesis of A83543N (Compound 3)

Using a procedure similar to that described in Example 5, A83543L (102.5 mg) was chemically converted to A83543N (66.5 mg).

### Example 12

### Preparation A83543AgA (Compound 17)

To a solution of A83543PsaA1 (6.03 g, 10.7 mmol) in methanol (267 ml), 7.2N H₂SO₄ (396 ml) was added, and the solution was heated to reflux for 3 hours. The mixture was then cooled in an ice bath. A large amount of NaHCO₃ (solid) and saturated aqueous NaHCO₃ were added cautiously; however, the pH was never brought above 1.0. The aqueous solution was mixed with ethyl ether and separated. The aqueous portion was then extracted with fresh ethyl ether. The ether extracts were combined, washed with brine, dried with K₂CO₃, and evaporated at reduced pressure. The resulting yellow semi-solid (4.89 g) was purified by normal phase chromatography with 100% dichloromethane and a gradient up to 7.5 percent methanol in dichloromethane, giving A83543AgA (2.83 g, 66 percent yield) as a colorless glass.

### Example 13

### Preparation of Compound 9

A suspension of N-chlorosuccinimide (104.7 mg, 0.78 mmol) in dichloromethane (2.6 ml) was cooled to -78°C under nitrogen. Diisopropyl sulfide (0.125 ml, 0.86 mmol) was added to this suspension, and the mixture was stirred at -78°C for one half hour. A83543J (184.6 mg, 0.26 mmol) in dichloromethane (1 ml) was then added slowly. When the addition was completed, the solution was stirred at -78°C for 6.25 hours. Triethylamine (0.109 ml, 0.78 mmol) was then added, and the solution was warmed to room temperature. The mixture was red. After warming, ethyl ether (6 ml) was added and a precipitate formed. The precipitate was dissolved in dichloromethane, and this was combined with the ethyl ether solution. The resulting solution was washed with 0.1N HCl, then washed with brine, dried with MgSO₄, and evaporated at room temperature. The resulting colorless glass (215 mg) was semi-purified by flash chromatography with 5 percent methanol in dichloromethane, giving Compound 9 as a colorless semi-solid (151.2 mg). The weight recovery and NMR spectrum showed contamination of product with diisopropyl sulfide, but the product was used without further purification.

### Example 14

### Preparation of Compound 10

The procedure used in Example 13 was repeated starting with A83543L (997.4 mg, 1.36 mmol), and gave Compound 10 as a colorless semi-solid (850 mg).

### Example 15

### Preparation of A83543PsaA2 (Compound 13)

To a solution of Compound 9 (1.89 g, 2.64 mmol) in methanol (100 ml), K₂CO₃ (anhydrous; 1.82 g, 13.2 mmol) was added, and the mixture was stirred at room temperature for one hour. Ethyl ether (100 ml) was then added and the mixture was filtered. The filtrate was evaporated at room temperature, giving a yellow solid. The yellow solid was dissolved in dichloromethane, washed with water, then brine, and dried with MgSO₄. The dichloromethane was then evaporated at reduced pressure, giving a colorless semi-solid (1.53 g). This semi-solid was purified by flash chromatography with 5 percent methanol in dichloromethane to 10 percent methanol in dichloromethane in a one-step gradient, giving A83543PsaA2 (1.09 g, 76 percent yield) as an off white glass.

### Example 16

### Preparation of A83543PsaD2 (Compound 14)

The procedure described in Example 15 was repeated using as starting material the product of Example 13 (770 mg, 1.06 mmol) and producing A83543PsaD2 (246 mg, 42 percent yield) as a colorless glass.

### Example 17

### Preparation of A83543AgD (Compound 18)

To a suspension of A83543PsaD2 (132 mg, 0.24 mmol) in water (5 ml), 1N H₂SO₄ was added dropwise until the mixture had a pH of 1.7 and was homogeneous. This solution was heated to 80°C for 3.75 hours, during which time an oil separated from the solution. The mixture was cooled to room temperature and dichloromethane was added to dissolve the oil. The aqueous layer was separated and extracted with fresh dichloromethane. The dichloromethane solutions were combined, washed quickly with 1N H₂SO₄, dried with K₂CO₃, and evaporated at room temperature giving a pale yellow glass (82.9 mg). The product was purified by flash chromatography with 5 percent methanol in dichloromethane, giving A83543AgD (63.6 mg, 63 percent yield) as a colorless glass.

Compounds 21 and 22 can be prepared by chemical demethylation of compounds 13 and 14, respectively, using sodium methoxide/iodine. The reaction is preferably carried out in a polar organic solvent, such as methanol. Further, the reaction is carried out at a temperature from about -10°C to about 15°C, preferably between 0°C and 5°C. The reaction times vary from about 4 hours to about 6 hours.

### Example 19

As indicated above, the compounds of this invention are useful as intermediates in the preparation of insecticides. For example, when appropriate microorganisms are cultured in the presence of the claimed compounds, the claimed compounds are bioconverted to insecticidally active A83543 components, as illustrated below.

This example illustrates the preparation of A83543A by culturing NRRL 18538 in the presence of A83543AgA. The culture *Saccharopolyspora spinosa* NRRL 18538, either as a lyophilized pellet or as a suspension maintained in liquid nitrogen, was used to inoculate a vegetative medium having the following composition:

| Ingredient | Amount (g) |
|---|---|
| Enzyme-hydrolyzed casein | 30 |
| Yeast extract | 3 |
| MgSO₄·7H₂O | 2 |
| Glucose | 10 |
| Deionized water | q.s. to 1 L |

The pH was adjusted to 6.5 with sodium hydroxide.

Slants or plates can be prepared by adding 2.5% agar to the vegetative medium. The inoculated slant is incubated at 30°C for 10 to 14 days. The mature slant culture is scraped with a sterile tool to loosen the spores and remove and macerate the mycelial mat. About one-fourth of the loosened spores and culture growth thus obtained is used to inoculate 50 ml of a first-stage vegetative medium. Alternatively, the first-stage medium may be inoculated from a liquid nitrogen ampoule.

When the culture is maintained in liquid nitrogen, ampoules are prepared using equal volumes of vegetative culture (48-72 hours incubation, 30°C) and suspending medium. The suspending medium contains lactose (100 g), glycerol (200 ml), and deionized water (q.s. to 1 L).

A liquid nitrogen ampoule is used to inoculate 100 ml of vegetative medium in 500 ml Erlenmeyer flasks (or 50 ml of medium in 250 ml flasks). The cultures are incubated at 30°C for 48 hours on a shaker orbiting in a two inch (5.08 cm) circle at 250 rpm. The incubated culture (5% v/v inoculum) is used to inoculate 100 ml of a production medium having the following composition:

| Ingredient | Amount (g) |
|---|---|
| Glucose | 80 |
| Peptionized milk | 20 |
| Cottonseed flour | 30 |
| Corn steep liquor | 10 |
| Ca₂CO₃ | 5 |
| Methyl oleate | 30 |
| Tap water | q.s. to 1L |

The pH was adjusted to 7.2 with sodium hydroxide.

Conversion of A83543AgA to A83543A was accomplished by addition of A83543AgA (4.88 mg, 0.195 mg/ml) to a 65 hr culture of NRRL 18538 in the above-mentioned production medium (25 ml in a 250 ml flask) and incubating the culture for an additional 31 hours. Acetonitrile (3.0 ml) was added to an aliquot (1.0 ml) of the culture. This sample was mixed and centrifuged and an aliquot was injected onto an analytical HPLC column designed to assay the various components of the A83543 culture. Analysis of the fermentation broth showed the presence of 2.44 mg (0.098 mg/ml) of A83543A.

## Claims

1. A compound of the Formula 1: 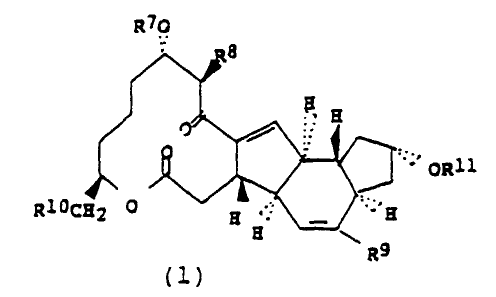 wherein R⁷, R⁸, R⁹, R¹⁰ and R¹¹ are each individually as follows:

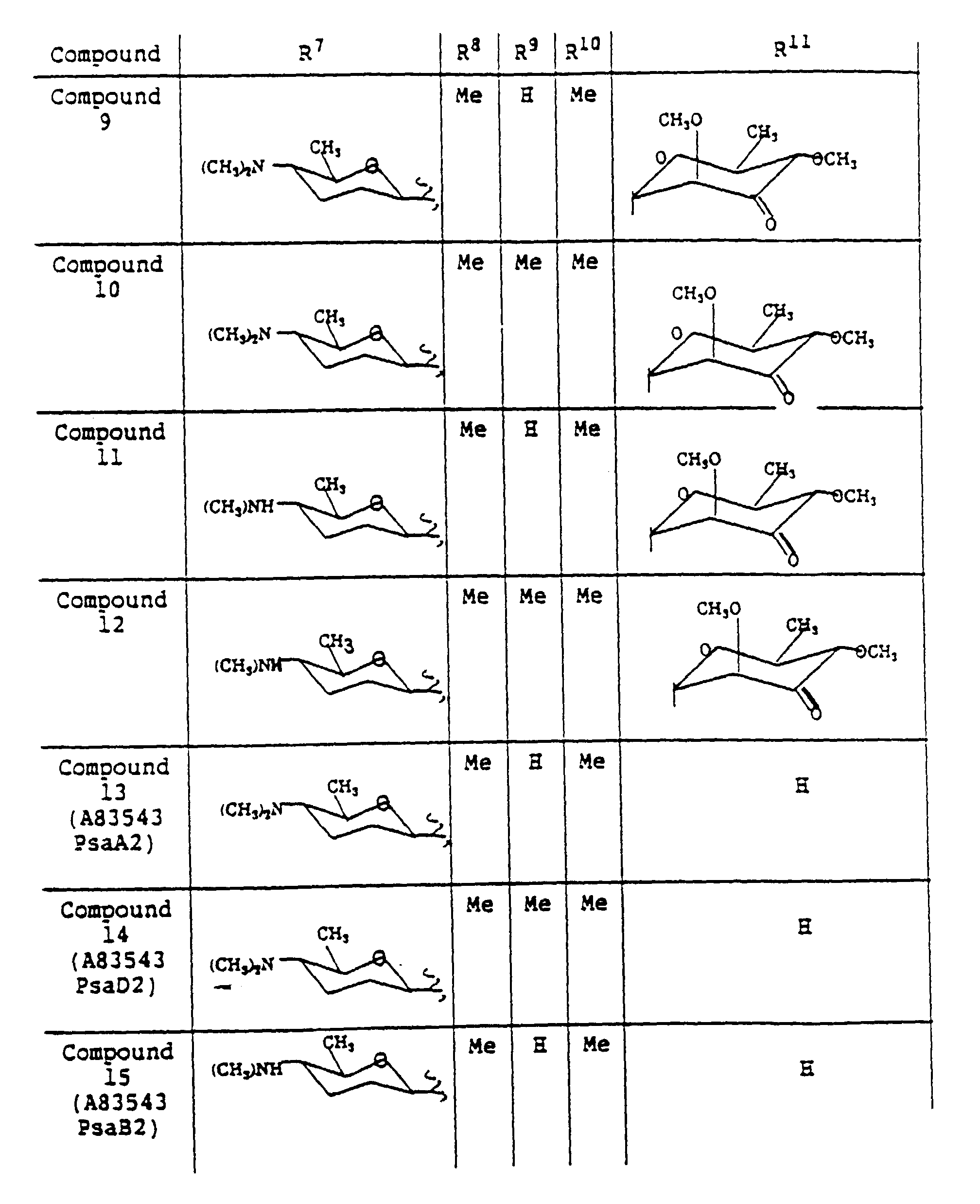

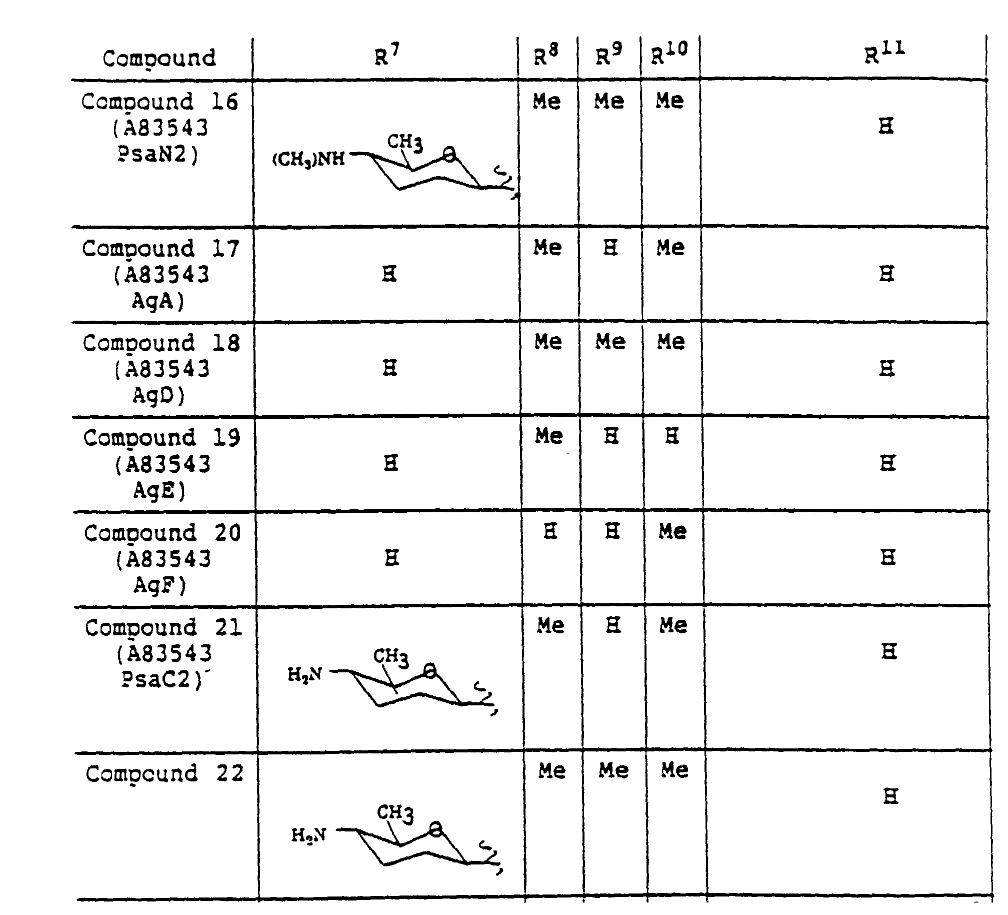

2. A process for preparing a compound 13, 14, 15, or 16 as defined in Claim 1, which process comprises removing the ketone-containing R¹¹ group from a corresponding compound 9, 10, 11, or 12 as defined in Claim 1.

3. A process as claimed in Claim 2, which process comprises oxidising a corresponding compound of formula 1 as defined in Claim 1, wherein R¹¹ is 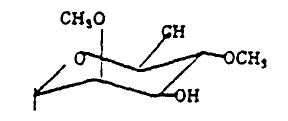 to produce the corresponding ketone-containing compound 9, 10, 11, or 12, and then removing the ketone-containing R¹¹ group from the said compound 9, 10, 11, or 12, to produce the corresponding compound 13, 14, 15, or 16.

4. A process for preparing a compound 21 or 22 as defined in Claim 1, which process comprises demethylating the corresponding compound 13 or 14 as defined in Claim 1.

5. A process for preparing a compound 9, 10, 11, or 12 as defined in Claim 1, which process comprises oxidising a corresponding compound of formula 1 as defined in Claim 1, wherein R¹¹ is

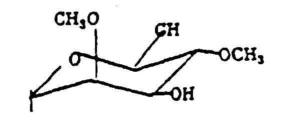

6. A process for preparing a compound 17, 18, 19, or 20 as defined in Claim 1, which process comprises
(a) hydrolyzing A83543A, A83543B, A83543C, A83543G, A83543H, A83543J, A83543PsaA1, A83543PsaA2, A83543PsaH1, A83543PsaJ1, A63543PsaB2, or A83543PsaC2, to produce A83543AgA; or
(b) hydrolyzing A83543PsaD2 or A83543PsaN2 to produce A83543AgD; or
(c) hydrolyzing A83543E or A83543PsaE1 to produce A83543AgE; or
(d) hydrolyzing A83543F or A83543PsaF1 to produce A83543AgF.

## Patentansprüche

1. Verbindung der Formel 1: 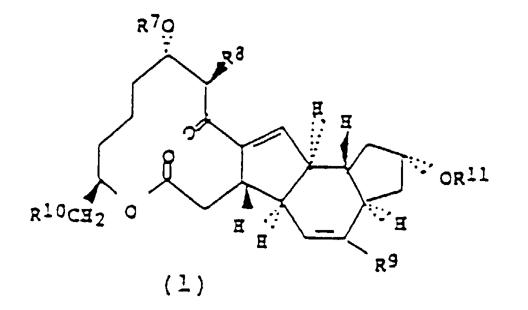 worin R⁷, R⁸, R⁹, R¹⁰ und R¹¹ jeweils unabhängig wie folgt sind:

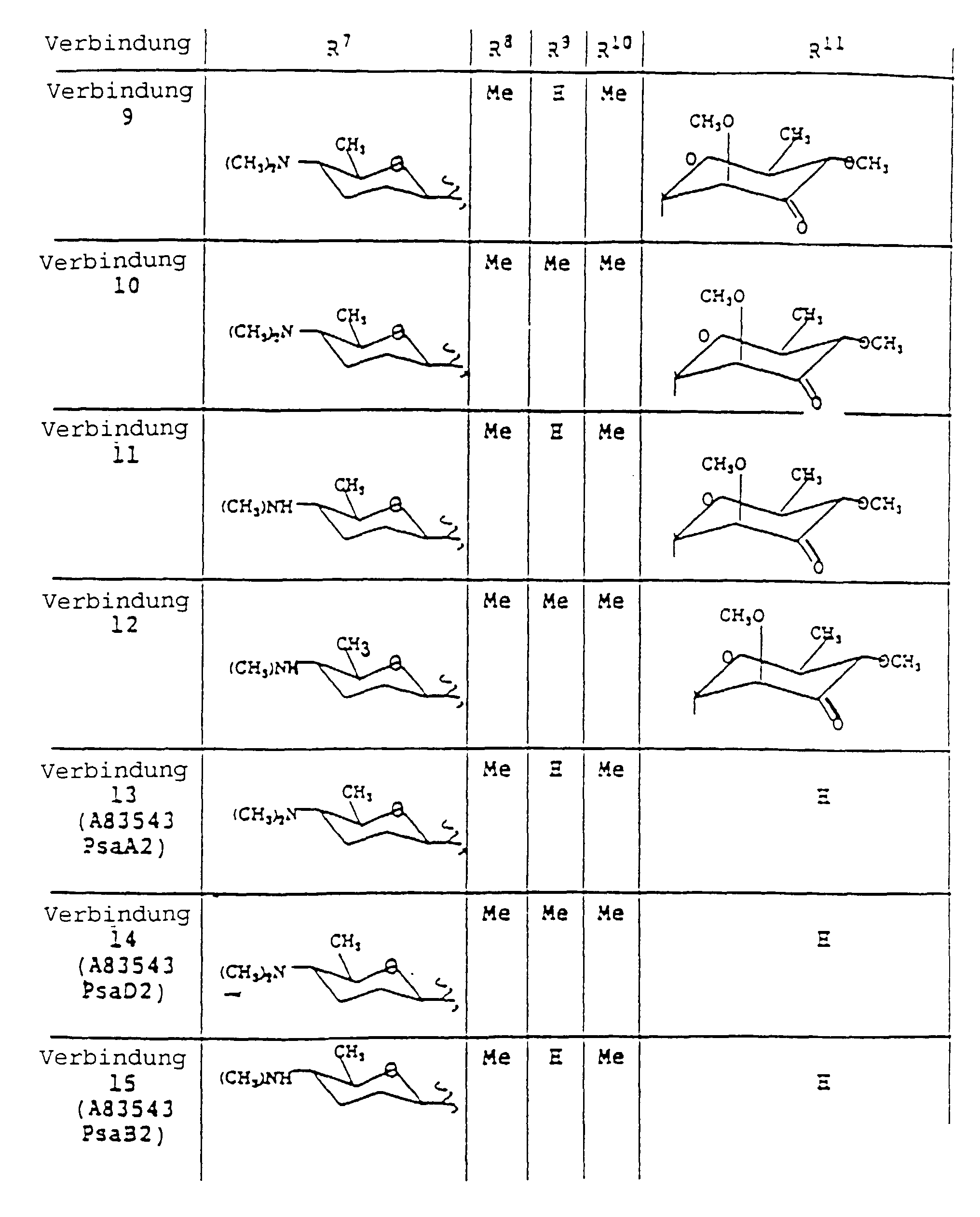

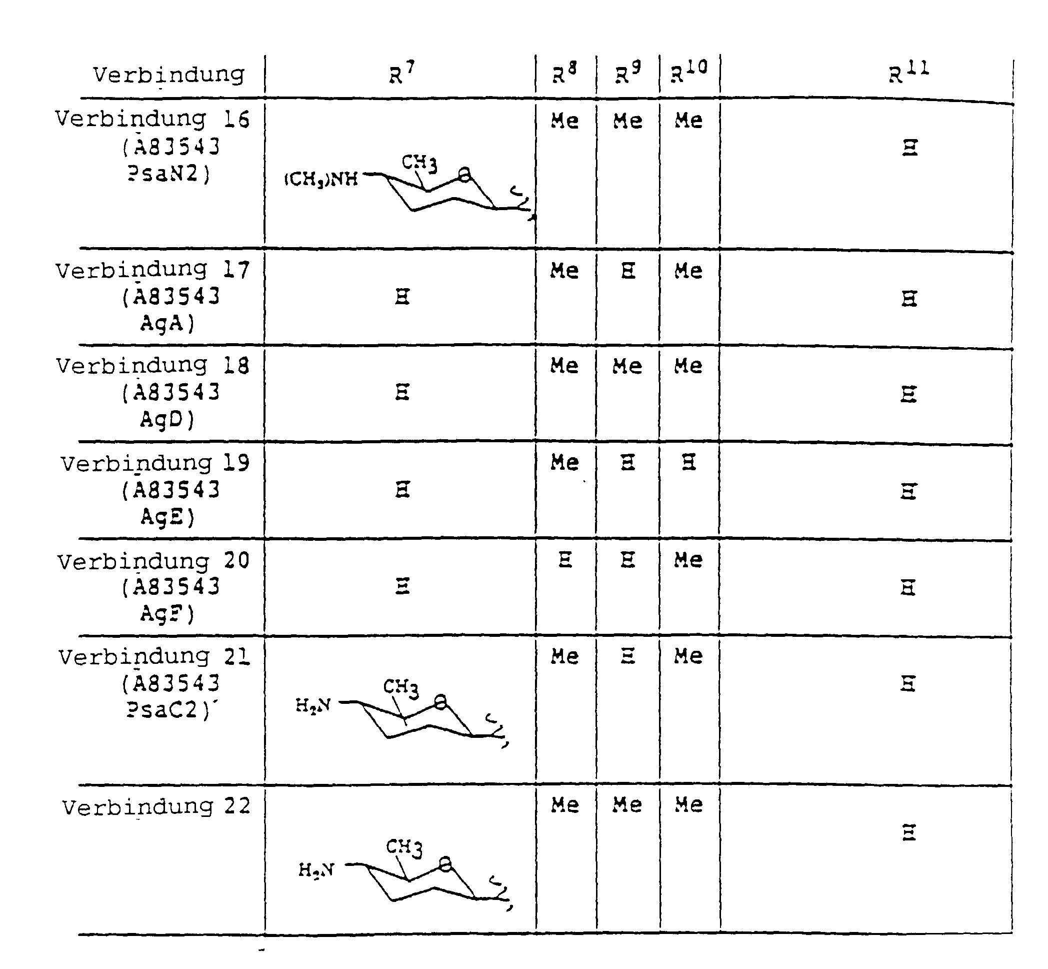

2. Verfahren zum Herstellen einer Verbindung 13, 14, 15 oder 16 nach Anspruch 1, wobei das Verfahren das Entfernen der Keton-enthaltenden R¹¹-Gruppe von einer entsprechenden Verbindung 9, 10, 11 oder 12 nach Anspruch 1 umfaßt.

3. Verbindung nach Anspruch 2, wobei das Verfahren das Oxidieren einer Verbindung der Formel 1 nach Anspruch 1 umfaßt, worin R¹¹ 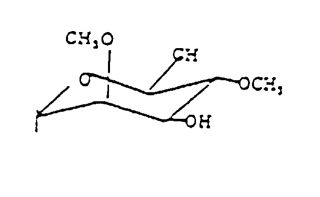 ist, um die entsprechende Keton-enthaltende Verbindung 9, 10, 11 oder 12 zu erzeugen, und dann Entfernen der Keton-enthaltenden R¹¹-Gruppe aus der Verbindung 9, 10, 11 oder 12, um die entsprechende Verbindung 13, 14, 15 oder 16 zu erzeugen.

4. Verfahren zum Herstellen einer Verbindung 21 oder 22 nach Anspruch 1, wobei das Verfahren das Demethylieren der entsprechenden Verbindung 13 oder 14 nach Anspruch 1 umfaßt.

5. Verfahren zum Herstellen einer Verbindung 9, 10, 11 oder 12 nach Anspruch 1, wobei das Verfahren das Oxidieren einer entsprechenden Verbindung nach Formel 1 nach Anspruch 1 umfaßt, worin R¹¹ 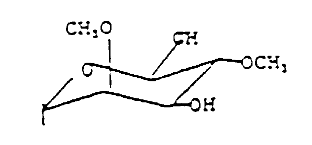 ist.

6. Verfahren zum Herstellen einer Verbindung 17, 18, 19 oder 20 nach Anspruch 1, wobei das Verfahren umfaßt:
(a) Hydrolysieren von A83543A, A83543B, A83543C, A83543G, A83543H, A83543J, A83543PsaA1, A83543PsaA2, A83543PsaH1, A83543PsaJ1, A83543PsaB2 oder A83543PsaC2, um A83543AgA zu erzeugen; oder
(b) Hydrolysieren von A83543PsaD2 oder A83543PsaN2, um A83543AgD zu erzeugen; oder
(c) Hydrolysieren von A83543E oder A83543PsaE1, um A83543AgE zu erzeugen; oder
(d) Hydrolysieren von A83543F oder A83543PsaF1, um A83543AgF zu erzeugen.

## Revendications

1. Composé de formule 1 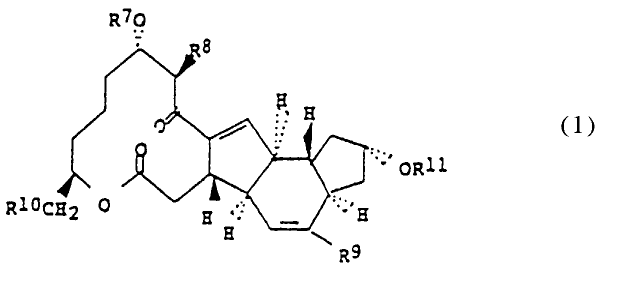 dans laquelle R⁷, R⁸, R⁹, R¹⁰ et R¹¹ ont chacun, individuellement, les significations suivantes :

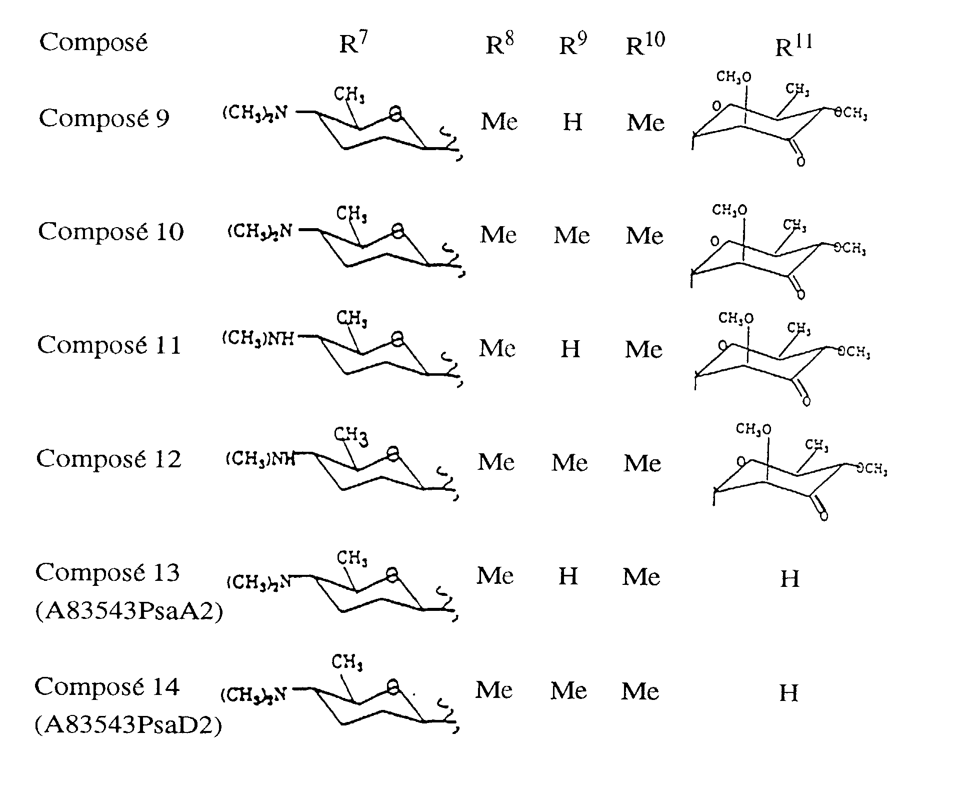

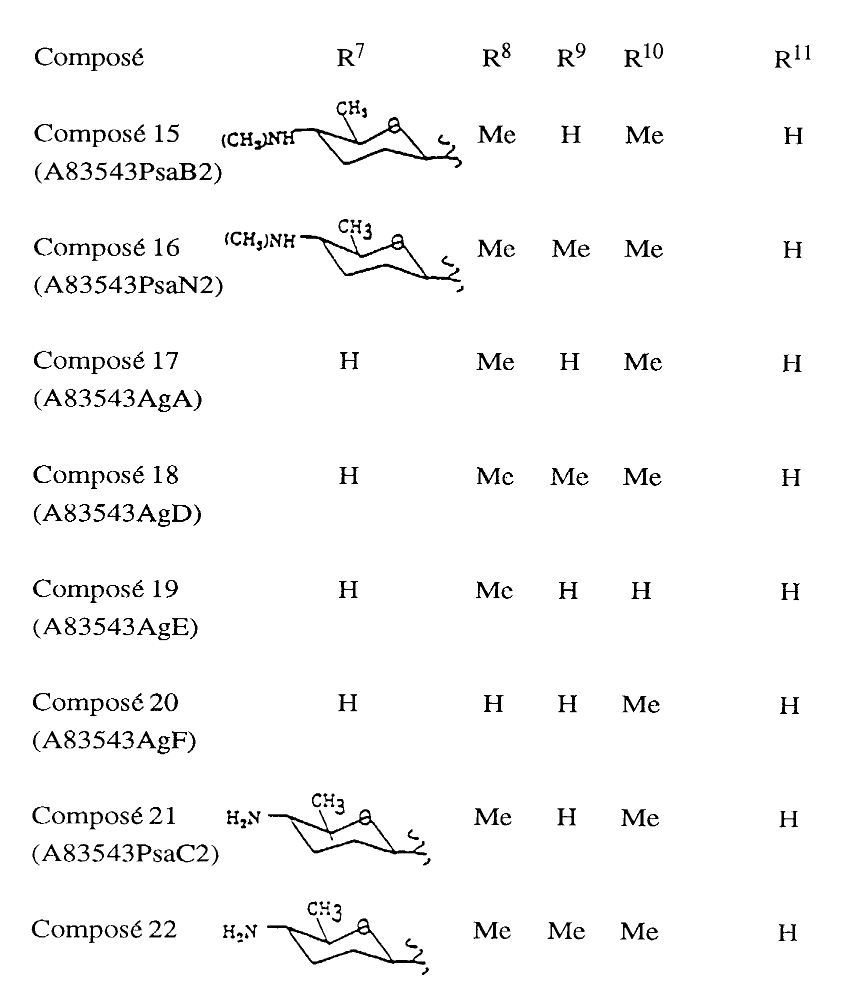

2. Procédé de préparation du composé 13, 14, 15 ou 16 défini dans la revendication 1, lequel procédé comporte le fait d'éliminer le groupe R¹¹ à fonction cétone du composé correspondant 9, 10, 11 ou 12 défini dans la revendication 1.

3. Procédé conforme à la revendication 2, lequel procédé comporte le fait d'oxyder un composé correspondant de formule 1, définie dans la revendication 1, où R¹¹ représente 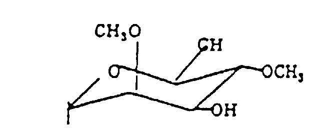 pour produire le composé correspondant 9, 10, il ou 12, à fonction cétone, et le fait d'éliminer ensuite le groupe R¹¹ à fonction cétone dudit composé 9, 10, il ou 12, pour produire le composé correspondant 13, 14, 15 ou 16.

4. Procédé de préparation du composé 21 ou 22 défini dans la revendication 1, lequel procédé comporte le fait déméthyler le composé correspondant 13 ou 14 défini dans la revendication 1.

5. Procédé de préparation du composé 9, 10, il ou 12 défini dans la revendication 1, lequel procédé comporte le fait d'oxyder un composé correspondant de formule 1, définie dans la revendication 1, où R¹¹ représente

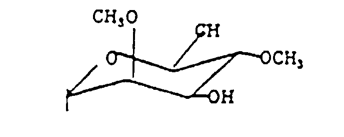

6. Procédé de préparation du composé 17, 18, 19 ou 20 défini dans la revendication 1, lequel procédé comporte
a) le fait d'hydrolyser le composé A83543A, A83543B, A83543C. A83543G, A83543H, A83543J, A83543PsaA1, A83543PsaA2, A83543PsaH1, A83543PsaJ1, A83543PsaB2 ou A83543PsaC2. pour produire le composé A83543AgA ; ou
b) le fait d'hydrolyser le composé A83543PsaD2 ou A83543PsaN2, pour produire le composé A83543AgD ; ou
c) le fait d'hydrolyser le composé A83543E ou A83543PsaE1. pour produire le composé A83543AgE ; ou
d) le fait d'hydrolyser le composé A83543F ou A83543PsaF1. pour produire le composé A83543AgF.
